# EUROPEAN PATENT APPLICATION

(11) **EP 2 075 026 A1**
(43) Date of publication of application: **01.07.2009**
(21) Application number: 07425826.0
(22) Date of filing: 28.12.2007
(51) Int. Cl.: A61M 16/08

(54) **Fluid delivery conduit and manufacturing method thereof**

(71) Applicant: Deas S.R.L., 48014 Castel Bolognese RA (IT)
(72) Inventor: Scardovi, Domenico, 48014 Castel Bolognese (Prov. of Ravenna) (IT)
(74) Representative: Alagem Modiano, Lara S.

(57) **Abstract**

A fluid conveyance duct (1) comprising a tubular element (2), at least one stiffening stem (3) and at least one conducting wire (4). The stem (3) and the conducting wire (4) are associated with the tubular element (2). The tubular element (2) comprises at least one first inner tubular layer (5) and at least one second outer tubular layer (6). At least one conducting wire (4), adapted to conduct electric current, light, heat and electromagnetic waves in general, is interposed between the first and second tubular layers (5, 6).

## Description

The present invention relates to a fluid conveyance duct and to the associated manufacturing method.

Such ducts are particularly widespread in the medical field for conditioning and adjusting the temperature and humidity of the fluid conveyed therein.

This control can occur directly or indirectly (for example, humidity can be controlled and managed as a consequence of temperature control) in order to monitor the characteristics of fluids such as air (in particular for respiratory ventilation but also for example for laparoscopy applications), solutions to be injected, blood or body fluids to be heated.

Known types of duct generally comprise a tubular element proper, of the flexible type, provided with suitable transverse stiffening means designed to avoid compression of the tube due to unwanted external actions.

These stiffening means are constituted generally by a stem, made of substantially rigid material, which is wound in a helical pattern onto the outer surface of the tubular element: the rigidity of the stem avoids crimping of the tubular element.

It is also known to provide such ducts with heating bodies associated with the surface of the ducts in order to control and manage the parameters of the conveyed fluid (in particular temperature and humidity).

The heating bodies are generally wires made of electrically conducting material which have a predefined electrical resistance value.

When these conducting wires are crossed by a predefined electric current, due to the Joule effect, they undergo a heating which, by conduction, is transferred to the tubular element.

The gas (or liquid) that flows through the duct, by striking the internal surface of the tubular element, is heated, further avoiding (with reference to applications for respiratory ventilation) the risk of forming condensation inside the duct.

According to a known version of this type of duct, the heating conducting wires are wound externally with respect to the tubular element.

In this case, the greater the thickness of the tubular element, the lower the effectiveness of the heating of the stream of fluid, since the heat must pass through the entire thickness, in practice heating also the entire volume of material that constitutes the tubular element. In practice, the heating conducting wires deliver most of the heat toward the outside environment (toward which they encounter less heat resistance) and therefore the efficiency of transmission toward the conveyed fluid is generally low.

Moreover, it must be noted that the patient is located proximate to the tube and therefore can be in direct contact with the outer surface of the duct, and therefore the heat emitted by the heating wires can inconvenience him and in some cases even cause pain.

A constructive solution is known which accommodates the conducting heating wires designed to heat the gas (or liquid) that flows within the duct inside the rigid stem wound helically onto the tubular element.

In this manner, the patient is never in direct contact with the insulating surface of the heating wires: the interposition of material avoids the onset of discomfort or pain which can be linked to the excessive temperature of the heating wires.

However, the rigidity of the stem makes it difficult to perform the wiring of the heating conducting wires, since the wires have to be extracted from the stem in order to be able to mate them with the respective power supply terminals. Extraction often requires a softening of the stem by means of heat sources and/or its breakage (of course without damaging the internal heating wires).

Secondarily, the insertion of the heating conducting wires within the stem entails a greater distance thereof from the internal surface of the tubular element, with consequent reduction of the efficiency of heat transmission to the flowing gas (or liquid).

The concept of arranging the heating conducting wires within the stem further allows only a distribution of the heating conducting wires which is wound with a predefined pitch equal to the winding pitch of the stem. It is therefore not possible to adopt more uniform distributions and/or distributions according to shorter pitches which would ensure more uniform and efficient heating.

If the duct is subjected to a particularly intense external action (impact or crimping), the duct can be crimped, possibly breaking the stiffening stem.

Since the stem is rigid and scarcely deformable, it can in fact have a mechanical behavior of the brittle type: the arrangement of the heating conducting wires within the stem can be risky, since an accidental fracture of the stem would entail damage to the wires and probably make it impossible to use the entire duct.

In any case, providing a duct in which the heating conducting wires are inserted in the stiffening stem entails complex and slow production cycles.

The aim of the present invention is to provide a fluid conveyance duct with high efficiency in heat transmission between the heating conducting wires and the fluid that flows inside the duct.

Within this aim, an object of the present invention is to provide a fluid conveyance duct in which the conducting wires can be easily deprived of the electrical insulation at the terminal ends for connection to a suitable power supply circuit.

Another object of the present invention is to provide a fluid conveyance duct in which the conducting wires are proximate to the internal surface of the tubular element.

Another object of the present invention is to provide a fluid conveyance duct in which the winding of the tubular surface by means of the conducting wires (be they conductors of electric current, heat and/or signal of any type, even optical) can be configured at will in order to ensure maximum uniformity of heating and conditioning of the fluid.

Another object of the present invention is to provide a fluid conveyance duct by means of a process which is quick, efficient and inexpensive.

Another object of the present invention is to provide a fluid conveyance duct by means of a continuous process in order to obtain indeterminate lengths of duct according to the invention.

A further object of the present invention is to provide a fluid conveyance duct which has low costs, is relatively simple to provide in practice and is safe in application.

This aim and these and other objects, which will become better apparent hereinafter, are achieved by the present fluid conveyance duct, of the type comprising a tubular element, at least one stiffening stem and at least one conducting wire, said stem and said conducting wire being associated with said tubular element, characterized in that said tubular element comprises at least one first inner tubular layer and at least one second outer tubular layer, at least one conducting wire, adapted to conduct electric current, light, heat and electromagnetic waves in general, being interposed between said first and second tubular layers.

Further characteristics and advantages of the invention will become better apparent from the following detailed description of a preferred but not exclusive embodiment of a fluid conveyance duct, illustrated by way of nonlimiting example in the accompanying drawings, wherein:

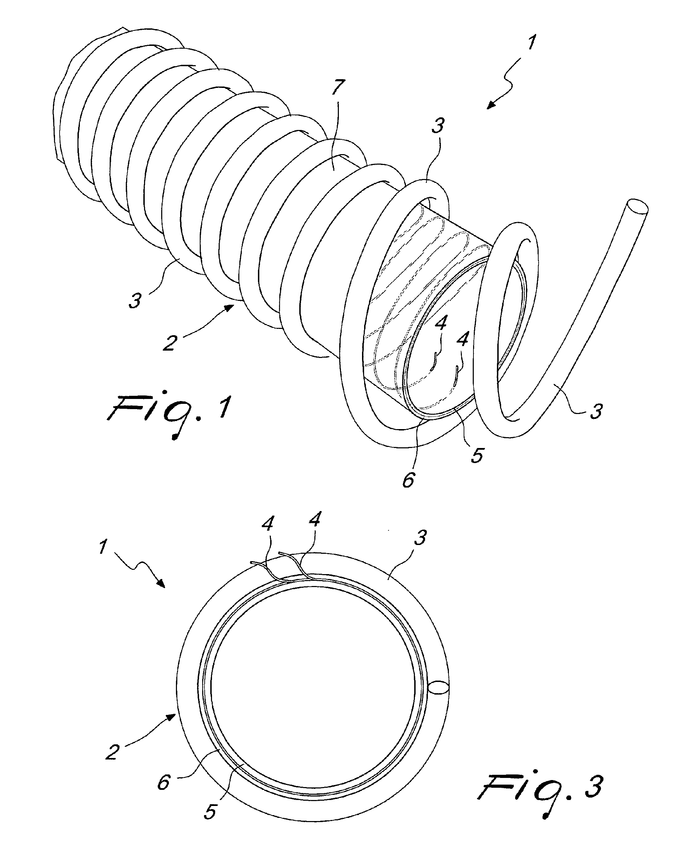

Figure 1 is a perspective view of a portion of a fluid conveyance duct according to the invention;

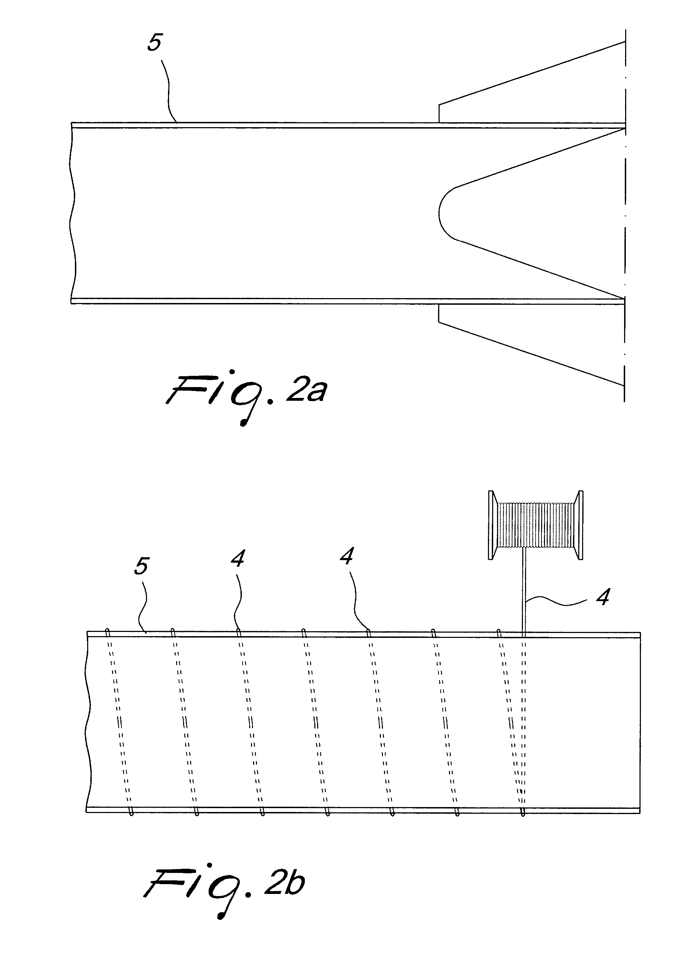

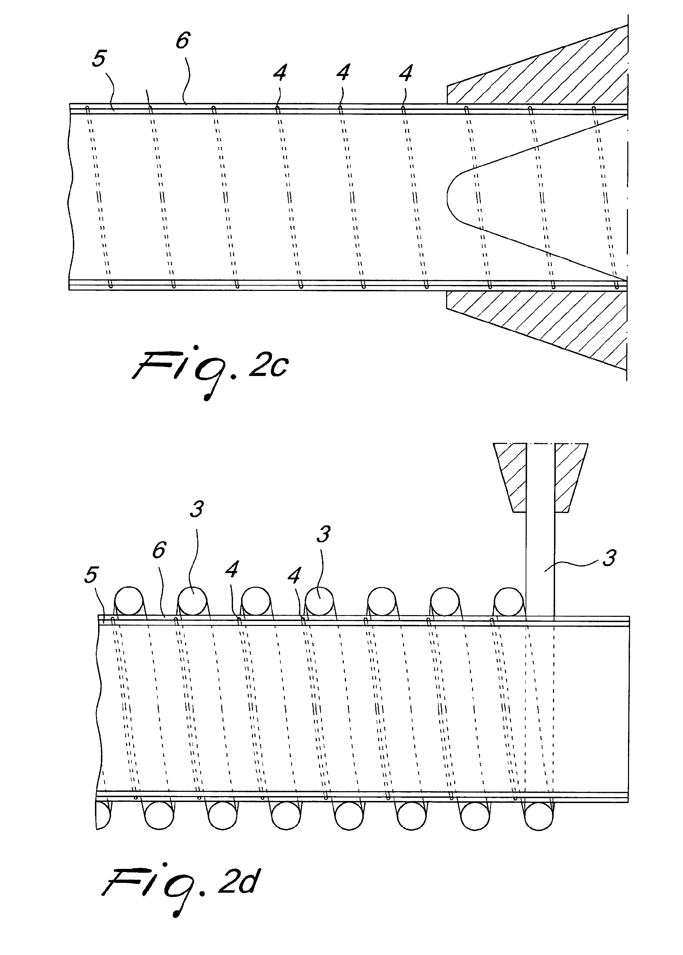

Figures 2a-2d are schematic views of the method for manufacturing a fluid conveyance duct according to the invention;
Figure 3 is a schematic front view of a possible embodiment of a fluid conveyance duct according to the invention.

With reference to the figures, the reference numeral 1 generally designates a fluid conveyance duct.

The duct 1 comprises a tubular element 2 on which at least one stiffening stem 3 and at least one conducting wire 4 are arranged substantially with a helical configuration (although any type of distribution is possible).

The tubular element 2 comprises at least one first inner tubular layer 5 and at least one second outer tubular layer 6.

The conducting wire 4 is interposed between the first tubular layer 5 and the second tubular layer 6: in particular, the conducting wire 4 is accommodated along the interface surface between two contiguous tubular layers.

In any one of the possible embodiments of the duct 1 according to the invention, the stiffening stem 3 is always arranged along an external surface 7 of the outermost outer tubular layer, with a substantially helical distribution.

According to an embodiment of particular interest in practice and in application, the conducting wire 4 is of the heating type.

In practice, it is a conducting wire 4 made of a material which has a preset electrical conductivity, in order to determine a total value of electrical resistance which is adapted to ensure the correct application of heat by Joule effect when it is crossed by a preset value of electric current.

The arrangement on the surface of discontinuity (interface) between the two contiguous layers 5 and 6 ensures extreme proximity of the heating conducting wires 4 to the internal surface of the inner tubular layer 5: this surface is struck by the stream of gas (or liquid) conveyed by the duct 1 and therefore the proximity of the heating wires 4 thereto ensures optimum efficiency in the transmission of heat to the conveyed gas (or liquid).

With respect to the duct 1, the stiffening stem 3 and the heating wire 4 are distributed respectively according to a first helical configuration and according to a second helical configuration, which have mutually independent geometric parameters.

In particular, this allows to distribute the heating wire 4 with a pitch at will, ensuring the obtainment of uniform heating of the duct 1: by using a particularly tight pitch it is in fact possible to achieve substantial continuity in the temperature values that can be detected on the internal surface of the innermost layer 5 and therefore a heating (and associated humidity control) of the conveyed fluid.

Independently of the choices made for the heating wire 4, the stiffening stem 3 can thus have an extremely tight pitch. Said pitch can make the stem 3 substantially cover completely the outer surface of the outer layer 6, forming in practice a new smooth and even surface.

The duct 1 according to the invention comprises a cable for transferring signals from suitable units arranged upstream of the duct 1 to suitable assemblies arranged downstream of the duct 1. Said cable, which can be interposed between the first tubular layer 5 and the second tubular layer 6, can be an electrical cable, or an optical fiber, or any constructive solution suitable for the indicated purpose. The cable can also be arranged on the outer surface 7 of the outermost outer tubular layer or on the inner surface of the innermost inner tubular layer.

In particular, sensors intended to detect the characteristics of the fluid when it is introduced in the respiratory system of the patient (with reference to the embodiment for respiratory ventilation, which is an application of particular practical interest) and other sensors and probes directly associated with the patient to monitor his vital parameters are installed downstream of the duct 1. These sensors and probes, by means of the signal transfer cable, are sent to a management and control unit, which as a function of said data performs suitable control of the temperature of the gas (or liquid) that flows (by means of the adjustment of the intensity of the current circulating on the heating wire 4) and makes these operations visible to the personnel by means of respective screens (or displays).

The at least one first inner tubular layer 5 and the at least one second outer tubular layer 6 are made of polymeric material: in particular, they can be provided by means of the traditional polymers used in the medical field. It must be stressed that it is possible to adopt superimposed layers (any chosen number of layers) in which the inner layer 5 is made of polymeric material of the type normally used in the medical field; the other layers, by not coming into contact with the gas (or liquid) that flows through the duct 1, can be made of any material.

It is even possible to provide the at least one first inner tubular layer 5 by using material with high heat conductivity and the at least one second outer tubular layer 6 by using material which has a lower heat conductivity than the material of the at least one first layer 5. The conducting wire 4, if wound with a sufficiently tight pitch, can also constitute itself a reflective surface which reduces heat radiation toward the outer layers. As an alternative, it is easily possible to interpose between two contiguous layers a sheet of reflective material which is designed to increase thermal insulation (reducing the effect of radiation).

An embodiment in which the at least one second outer tubular layer 6 has an internal surface, for interfacing with the corresponding outer surface of the at least one first inner tubular layer 5 which faces it and is proximate thereto, of the reflective type in order to minimize outward heat transmission by radiation, is interesting.

From the constructive standpoint, the duct 1 is constituted by the at least one first inner tubular layer 5 and the at least one second outer tubular layer 6, which form a respective interface surface along which they are mutually welded. The mutual weld is rather weak: thanks to the limited bonding strength of the two layers 5 and 6, said layers can be separated easily for facilitated access to the at least one conducting wire 4. Convenience of access to the conducting wire 4 is particularly interesting, since it allows and facilitates the operations for preparing the wire 4 for its connection to the power supply circuits and/or to the components with which it is to be associated. In particular, each wire 4 of the type of enameled copper has its own surface electrical insulation which must be removed, and therefore ease of access makes this operation and all the wiring operations easy and simple.

According to an embodiment of particular interest in practice and in application, the conducting wires 4 and/or the signal transfer cables are a plurality; they are arranged along the plurality of interface surfaces formed between pairs of superimposed and mutually facing tubular layers.

In addition to all the applications in which the wire 4 is of the heating type, it is in fact convenient to note that it is possible to arrange at least one conducting wire 4 and at least one signal transfer cable along the interface surfaces formed between contiguous tubular layers: the separation of different wires 4 on different interface surfaces ensures that the mutual insulation between all the wires 4 that are present is effective, also reducing noise caused by mutual induction.

For example, it is possible to arrange along each interface surface both a respective wire 4 and a corresponding cable, or it is possible to arrange a wire 4 on the innermost interface surfaces, so as to better ensure the heating of the fluid, while the cables are arranged on the outer interface surfaces.

The method for providing the duct 1 for conveying fluids consists in extruding a first inner tubular layer 5: the extruder is therefore provided with a first extrusion head whose diameter is consistent with the diameter sought for the first layer 5 and works exclusively on materials of the polymeric type used traditionally in the medical field (or other materials that have similar chemical and physical characteristics).

It is therefore necessary to arrange at least one conducting wire 4 along the outer surface of the inner tubular layer 5: said wire is generally wound with a helical pattern whose pitch depends on the function that must be performed by the wire 4.

Finally, it is necessary to extrude a second outer tubular layer 6 over the outer surface of the first inner tubular layer 5 surmounted by the at least one conducting wire 4: a second extrusion head (whose dimensions are consistent with those intended for the layer 6 being provided) performs this step.

Since it can be convenient to provide ducts 1 constituted by a plurality of superimposed layers, at the end of the step for extruding the second outer tubular layer 6 it is necessary to arrange at least one second conducting wire 4 (which has, for example, different functions with respect to the one wound previously between the layers located further inward) along the outer surface of said layer and extrude, over said outer surface of the layer, surmounted by said at least one second conducting wire, an additional outer tubular layer.

In order to provide a duct 1 which is particularly resistant with respect to external crimping actions, at the end of the step for extrusion of the outer tubular layer 6 it is necessary to arrange at least one stiffening stem 3 along the outer surface 7 of the outermost tubular layer 6.

It should be noted that the arrangement of the at least one conducting wire 4 and the arrangement of the at least one stiffening stem 3 occur along substantially independent distributions. It is even possible to use different arrangement criteria for each individual wire 4 arranged in the duct 1 and for each individual stem 3.

It is also possible to arrange an additional layer over the outer layer on which the stem 3 is arranged and to place on the new outer surface an additional stem 3 with a distribution which is independent of the one of the inward stem 3.

It has thus been shown that the invention achieves the intended aim and objects.

The invention thus conceived is susceptible of numerous modifications and variations, all of which are within the scope of the appended claims.

As an alternative to the mentioned external distribution of the stem 3, it is possible to consider an embodiment in which at least one tubular layer with protective and/or thermal insulation functions is extruded (or arranged with any other method) over the stem 3.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A fluid conveyance duct, of the type comprising a tubular element (2), at least one stiffening stem (3) and at least one conducting wire (4), said stem (3) and said conducting wire (4) being associated with said tubular element (2), **characterized in that** said tubular element (2) comprises at least one first inner tubular layer (5) and at least one second outer tubular layer (6), at least one conducting wire (4), adapted to conduct electric current, light, heat and electromagnetic waves in general, being interposed between said first and second tubular layers (5, 6).

2. The duct according to claim 1, **characterized in that** said stiffening stem (3) is arranged along the outer surface (7) of the outermost at least one outer tubular layer (6) according to a first substantially helical configuration.

3. The duct according to claim 1, **characterized in that** said at least one conducting wire (4) is of the heating type, being interposed between said at least one first tubular layer (5) and said at least one second tubular layer (6), said at least one conducting wire (4) being arranged along a second substantially helical configuration.

4. The duct according to claims 2 and 3, **characterized in that** said first substantially helical configuration and said second substantially helical configuration have mutually independent geometric parameters.

5. The duct according to claim 1, **characterized in that** it comprises at least one cable for transferring signals from suitable units arranged upstream of said duct (1) to suitable assemblies arranged downstream of said duct (1), said at least one cable being interposed between said first and second tubular layers (5, 6).

6. The duct according to claim 1, **characterized in that** said at least one first inner tubular layer (5) and said at least one second outer tubular layer (6) are made of polymeric material.

7. The duct according to claim 1, **characterized in that** said at least one first inner tubular layer (5) is made of material with high thermal conductivity and said at least one second outer tubular layer (6) is made of a material which has a lower thermal conductivity than the material of the at least one first layer (5).

8. The duct according to claim 1, **characterized in that** said at least one second outer tubular layer (6) has an internal surface, for interfacing with the corresponding outer surface of the at least one first inner tubular layer (5) that faces it and is proximate thereto, which is of the reflective type in order to minimize the outward transmission of heat by radiation.

9. The duct according to one or more of the preceding claims, **characterized in that** said at least one first inner tubular layer (5) and said at least one second outer tubular layer (6) form a respective interface surface along which they are mutually welded, said weld being weak and said layers (5, 6) being therefore easily detachable for facilitated access to said at least one conducting wire (4).

10. The duct according to one or more of the preceding claims, **characterized in that** said conducting wires (4) are a plurality and are arranged along the plurality of interface surfaces formed between pairs of superimposed and mutually facing tubular layers.

11. The duct according to one or more of the preceding claims, **characterized in that** said at least one conducting wire (4) and said at least one signal transfer cable are arranged along said interface surfaces formed between said tubular layers.

12. A method for manufacturing a fluid conveyance duct (1) which consists in
- extruding a first inner tubular layer (5);
- arranging at least one conducting wire (4) along the outer surface of said inner tubular layer (5);
- extruding a second outer tubular layer (6) over said outer surface of said first inner tubular layer (5) surmounted by said at least one conducting wire (4).

13. The method according to the preceding claim, **characterized in that** at the end of the step for extrusion of said second outer tubular layer (6) it is necessary to arrange at least one second conducting wire (4) along the outer surface (7) of said layer (6) and extrude an additional outer tubular layer over said outer surface of the layer that is surmounted by said at least one second conducting wire (4).

14. The method according to the preceding claim, **characterized in that** at the end of the step for extrusion of said outer tubular layer (6) it is necessary to arrange at least one stiffening stem (3) along the outer surface of said outermost tubular layer (6).

15. The method according to claim 12, **characterized in that** the arrangement of the at least one said conducting wire (4) and the arrangement of the at least one stiffening stem (3) occur according to configurations which have mutually independent geometric parameters.
